# EUROPEAN PATENT APPLICATION

(11) **EP 3 569 221 A1**
(43) Date of publication of application: **20.11.2019**
(21) Application number: 19175178.3
(22) Date of filing: 17.05.2019
(51) Int. Cl.: A61K 8/41, A61K 8/73, A61K 31/46, A61Q 15/00, A61P 17/00, A61K 9/00, A61K 47/38

(54) **AQUEOUS FORMULATIONS COMPRISING IPRATROPIUM FOR TOPICAL TREATMENT OF HYPERHIDROSIS**

(30) Priority: 17.05.2018 EP 18172931
(71) Applicant: Notoxins IP B.V., 9801 VV Zuidhorn (NL)
(72) Inventor: Venema, Wim, 9801 VV Zuidhorn (NL); Homan, Ben, 9801VV Zuidhorn (NL)
(74) Representative: Nederlandsch Octrooibureau

(57) **Abstract**

The present invention relates to the field of topical aqueous formulations comprising ipratropium for therapeutically or cosmetically treating a skin disease.

## Description

### TECHNICAL FIELD OF THE INVENTION

The present invention relates to the field of topical aqueous formulations comprising ipratropium for therapeutically or cosmetically treating a skin disease.

### BACKGROUND OF THE INVENTION

Sweating, or perspiring, is an essential and natural biological process for the thermoregulation of the human body. Sweating occurs in response to the body's temperature increase due to physical exertion or environmental conditions, but may be also triggered by emotions. Although odorless, the sweat produced by the sweat glands may become beyond inconvenient due to its breakdown by bacteria living on the skin which results in the production of unpleasant odours.

Focal or generalized hyperhidrosis, a clinical condition characterized by excessive sweating, occurs typically during puberty or adult life. Excessive sweating makes unpleasant odours formation more difficult to control. The affected individuals may become stigmatized and even be led to social isolation since the presence of excessive sweat (and its resulting odor) may be seen as evidence of insecurity and/or insufficient hygiene.

Deodorants and antiperspirant compositions are available to keep the skin (especially underarms) dry and odorless. Most of the compositions available in the market comprise astringent salts of aluminium and/or zirconium, such as aluminium chlorohydrate. These materials function by blocking the sweat ducts thereby reducing the production of perspiration. However, skin inflammation or allergy to such components is often reported.

WO 2010/062930 describes compositions and methods for ameliorating, preventing, and/or treating hyperhidrosis and/or excessive perspiration by topically applying a composition comprising an anticholinergic compound (e.g., an ipratropium compound) to a mammal. The examples describe lotions, creams, gels and ointments comprising 2.59 wt.% ipratropium. Examples 12 discloses a gel comprising 2.59 wt.% ipratropium bromide, 3.0 wt.% methylcellulose 1500 cps and 94.41 wt.% water.

There is a need for a pharmaceutical composition that is effective in the treatment of hyperhidrosis even at low concentration of ipratropium (reducing the associated costs with the treatment). In particular, there is a need for a simple treatment that provides instant relief of hyperhidrosis symptoms.

It is therefore desirable to provide ipratropium dosage forms that can be administrated topically and provide instant relief of hyperhidrosis symptoms.

### SUMMARY OF THE INVENTION

In a first aspect, the present invention relates to an aqueous formulation for use in the treatment of a skin disease, the treatment comprising topically administering to a human patient an aqueous formulation comprising, by weight of the total formulation:
- 0.01- 1.5wt.% ipratropium equivalent;
- 70-98 wt.% aqueous phase;
- dermatologically acceptable pH adjustment agent to provide the formulation with a pH in the range of 3.0 to 6.0 at 21°C.

It was unexpectedly found that the aqueous formulation of the present invention can be administered topically to provide instant relief of a skin disease, for example, hyperhidrosis symptoms, notably excessive perspiration. Without wishing to be bound by theory, the inventors postulate that the acidic pH of the formulation facilitates rapid and effective binding of ipratropium to the muscarinic receptors within the skin, e.g. the muscarinic receptors located on the sweat glands.

The therapeutic or cosmetic treatment using the aqueous formulations according to the invention is advantageously cheaper due to the low amounts of ipratropium, less allergenic since the ant perspiration effect does not depend on aluminium-derived components and other known skin irritating compounds, yet showing an excellent efficacy.

The present invention further relates to a skin applicator comprising the aqueous formulation of the invention, method of preparing the aqueous formulation of the invention and to a cosmetic treatment using the aqueous formulation of the invention.

### DETAILED DESCRIPTION OF THE INVENTION

In a first aspect, the present invention relates to the present invention relates to an aqueous formulation for use in the treatment of a skin disease, the treatment comprising topically administering to a human patient an aqueous formulation comprising, by weight of the total formulation:
- 0.01- 1.5wt.% ipratropium equivalent;
- 70-98 wt.% aqueous phase;
- dermatologically acceptable pH adjustment agent to provide the formulation with a pH in the range of 3.0 to 6.0 at 21°C.

The term "treatment" as used herein encompasses both therapeutic and palliative treatment of a human patient. In other words, by "treatment" is meant that at least an amelioration of the symptoms or characteristics associated with a condition afflicting the subject is achieved.

The term "hyperhidrosis" as used herein refers to a skin disease characterized by excessive perspiration in localized parts of the body or generalized excessive perspiration as diagnosed by a medical practitioner or reported by a subject.

Excessive perspiration may be quantitatively determined by gravimetric assessments, on the Hyperhidrosis Disease Severity Scale (HDSS), by measurement by transdermal epidural water vapor loss (e.g., Vapometer, Delfin Technologies, Kuopio Finland), on the Hyperhidrosis Visual Quantification Scale (HHVQS, e.g., HHVQSa or HHVQSp), on the Hyperhidrosis Visual Analog Scale (HHVAS) or any combination thereof.

The term "topical administration" as used herein refers to both epicutaneous administration (*i.e.*, the route of topical drug administration by which ipratropium is taken into the body via direct skin application) and local pharmacodynamic effect. In other words, the formulation of the invention is meant to be applied directly to or on the skin for local absorption and action of ipratropium. Accordingly, the "topical administration" used herein does not represent a significant route for systemic delivery of ipratropium.

"Ipratropium equivalent" as used herein refers to the substance (8-methyl-8-propan-2-yl-8-azoniabicyclo[3.2.1]octan-3-yl) 3-hydroxy-2-phenylpropanoate. Preferably, ipratropium as used herein refers to anhydrous or monohydrate ipratropium bromide. Typically, monohydrate ipratropium bromide is freely soluble in water (10 mg/mL) at 20 °C.

The term "aqueous phase" as used herein refers to an aqueous phase comprising water or pharmaceutically acceptable water-soluble components, such as ethanol, propylene glycol, glycerol, and conventional water-soluble components.

The "aqueous phase" is in the liquid state or in the semi-solid state at 20 °C.

The term "dermatologically acceptable pH adjustment agent" as used herein refers to buffering agents, salts of weak organic or inorganic acids, or organic or inorganic acids or bases..

The pH of the aqueous formulation according to the invention is measured at 21°C using conventional techniques known to the skilled person.

Suitable examples of aqueous formulations according to the invention comprise solutions, lotions, dispersions, suspensions, emulsions, foamable liquids, foams, aqueous gels, and the like.

The term "hyperhidrosis" as used herein refers to a skin disease characterized by excessive perspiration in localized parts of the body or generalized excessive perspiration as diagnosed by a medical practitioner or reported by a subject.

Excessive perspiration may be quantitatively determined by gravimetric assessments, on the Hyperhidrosis Disease Severity Scale (HDSS), by measurement by transdermal epidural water vapor loss (e.g., Vapometer, Delfin Technologies, Kuopio Finland), on the Hyperhidrosis Visual Quantification Scale (HHVQS, e.g., HHVQSa or HHVQSp), on the Hyperhidrosis Visual Analog Scale (HHVAS) or any combination thereof.

The terms "sweating" and "perspiring" are used herein as synonyms and refer to the biological act of fluid secretion by the eccrine and/or apocrine glands in a patient in response to nerve stimulation, emotional state, environmental conditions (*i.e.*, hot air temperature), and/or exercise.

The term "dry touch" as used herein refers to the cosmetic aspect of the skin such as a dry feel and an aesthetics sensation perceived by the subject upon alleviation or prevention of sweating or perspiration in normal subjects, *i.e.*, not in subjects suffering from hyperhidrosis.

The term "deodorant", as used herein, relates to personal care products that are applied topically to minimize the odor caused by the bacterial breakdown of perspiration. The term "antiperspirant", as used herein, refers to the subclass of deodorant products whose primary function is to inhibit perspiration, and normally act to the sweat glands level.

The term "water soluble" as used herein, unless indicated otherwise, refers to materials having a solubility in demineralized water of 21°C of more than 20 g/l.

According to an embodiment of the invention, ipratropium is present in the aqueous formulation in an amount of 0.3 to 1.5 wt.%, preferably in an amount of 0.5 to 1.0 wt.%.

Typically, ipratropium is present in the aqueous formulation in the form of a pharmaceutically acceptable salt. The pharmaceutically acceptable salt is suitably selected from ipratropium bromide anhydrous or monohydrate, or ipratropium chloride. Preferably, ipratropium is ipratropium bromide, more preferably ipratropium bromide monohydrate.

According to a preferred embodiment, the aqueous phase comprises, based on the weight of the aqueous phase, at least 50 wt.% water, preferably at least 60 wt.% water, more preferably at least 75 wt.% water, even more preferably at least 80 wt.% water.

In a preferred embodiment, the aqueous formulation is a liquid formulation selected from a lotion or a solution.

In another preferred embodiment, the aqueous formulation is an aqueous gel.

Ipratropium is substantially water-soluble in the aqueous phase of the aqueous formulations. Substantially water-soluble as used herein refers to a solubility of at least 90% in water when ipratropium in a concentration of 20 mg/ml is added to a buffered aqueous solution having pH 4 at 20 °C. Accordingly, in a most preferred embodiment, the ipratropium is substantially soluble in the aqueous formulation.

Improved results of the aqueous formulation according to the invention are observed when the aqueous formulation has a pH in the range of 3.5 to 6.0, more preferably 4.0 to 5.5, even more preferably 4.5 to 5.0. Without wishing to be bound by theory, it is believed that the increased efficacy of the aqueous formulation having acidic pH is due to the acidic pH of the sweat glands (about 5.0). Advantageously, a composition having a pH below 6 reduces the risk of irritation and smelling when applied to the skin, whilst presenting excellent stability.

The pH of the formulation is suitably adjusted by adding a dermatologically acceptable pH adjustment agent selected from buffering systems or salts of weak organic or inorganic acids, such as carbonate buffers, citrate buffers, phosphate buffers, acetate buffers, hydrochloric acid, lactic acid, tartric acid, diethylamine, triethylamine, diisopropylamine, aminomethylamine, or the like. Preferably, the buffer is citrate buffer.

If necessary, the pH of the final solution may be adjusted by adding suitable amounts of pH agents such as a neutralizing agent or an acidifying agent. Neutralizing agents are, for example, a ternary amine such as monoethanolamine, diethanolamine, triethanolamine, tromethamine, tetrahydroxypropylethylendiamine, aminomethyl propanol, diisopropanolamine, or an inorganic alkali such as NaOH solution, KOH solution, or NH₄OH solution. Acidifying agents are, for example, hydroxyacids solutions, such as a HCI solution, lactic acid solution, citric acid solution, phosphoric acid solutions, and the like.

The skilled person will appreciate that the concentration of dermatologically acceptable pH adjustment agent in the aqueous formulation may be adjusted without difficulty to provide an aqueous formulation having a pH in the range of 3.0 to 6.0, in view of the optional components present in the composition.

The aqueous formulation may suitably comprise 0.001 to 20 wt.% of dermatologically acceptable excipients. Typically, the excipients are selected from gelling agents, bactericides, preservatives, antioxidants, humectants, sequestering agents, moisturizers, emollients, surfactant, drying agents, fragrances and the like. More preferably, the aqueous formulation comprises 0.01 to 15 wt.%, even more preferably 0.05 to 10 wt.%, most preferably 0.5 to 5 wt.% of dermatologically acceptable excipients.

In a preferred embodiment, the formulation comprises 0.001 wt.% to 1 wt.% of one or more bactericides selected from benzalkonium chloride, triclosan, sorbic acid, citric acid or combinations thereof. In a particularly preferred embodiment, the formulation comprises 0.01 - 0.08 wt.%, more preferably 0.03 - 0.05 wt.% of benzalkonium chloride. The inventors have found that an aqueous formulation of ipratropium at an acidic pH has the advantage that it is very stable at 21°C for over 16 weeks in the presence of a preservative like benzalkonium or sorbic acid.

The aqueous formulations of the present invention preferably contains 0.1 to 30 wt.%, more preferably 0.5 to 20 wt.%, most preferably 1 to 10 wt.% of at least one cationic or non-ionic surfactant.

The inventors have further discovered that propylene glycol may adversely affect the stability and efficacy of the formulation if high amounts thereof is present. Accordingly, in a particularly preferred embodiment, the aqueous formulation comprises less than 15 wt.% propylene glycol, preferably less than 10 wt.%, more preferably less than 5 wt.% propylene glycol, even more preferably less than 3 wt.% propylene glycol by weight of the aqueous formulation. In a most preferred embodiment, the aqueous formulation does not comprise propylene glycol.

The aqueous formulation may comprise at least one gelling agent. In a preferred embodiment, the at least one gelling agent is present in an amount of at most 20.00 wt.%, more preferably 0.5 to 10 wt.%, even more preferably 1 to 5 wt.%. In a further preferred embodiment, the at least one gelling agent is present in an amount of at most 1.00 wt.%, more preferably at most 0.8 wt.%, even more preferably at most 0.5 wt.% by weight of the aqueous formulation.

Suitable gelling agents to be used in the aqueous formulation are cationic or non-ionic gelling agents, e.g., polyethylene glycol (PEG). Accordingly, the gelling agent preferably is not an anionic component, such as polyacrylate.

The aqueous formulation of the present invention preferably comprises a drying agent to provide improved dry skin feel in a concentration from about 0.1 % to about 5%, more preferably from about 0.3% to about 3%, even more preferably from about 0.5% to about 2%, by weight of the composition. Suitable drying agents are typically in the form of inert solid particulates, and include materials such as talc, microthene, silicates, colloidal silicas, and mixtures thereof. These materials provide benefits such as dry feel and aesthetics, especially dry feel benefits.

Preferably, the aqueous formulation according to the invention is for use in the treatment of primary hyperhidrosis or secondary hyperhidrosis, *i.e.*, in consequence of the use of certain medicaments or resulting from other diseases or conditions.

Primary hyperhidrosis usually develops during childhood and tends to persist throughout adult life. The main affected areas are the axilla, the palms of the hands, the soles of the feet, and the face (in particular the forehead), the back, or the belly.

Causes of secondary hyperhidrosis include endocrine diseases such as diabetes mellitus, hyperthyroidism, and hyperpituitarism, or neurological diseases including peripheral nerve injury, Parkinson's disease, reflex sympathetic dystrophy, spinal injury, and Arnold-Chiari malformation. Additional causes to consider are pheochromocytoma, respiratory diseases, and psychiatric diseases. The main affected areas are axilla, the palms of the hands, the soles of the feet, and the face (in particular the forehead), the back, or the belly.

Accordingly, the aqueous formulation according to the invention is preferably topically administered for the treatment of primary or secondary hyperhidrosis. More preferably, the primary or secondary hyperhidrosis is selected from palmar, plantar, axillary, facial, truncal, thigh or gustatory (lips, nose and forehead) hyperhidrosis or combinations thereof.

In accordance with a particularly preferred embodiment, the treatment comprises topically administering the aqueous formulation according to the invention to the affected skin to provide ipratropium in a dosage of 0.015 to 1.5 mg per cm² of skin, preferably 0.01 to 1.0 mg per cm² of skin, even more preferably 0.005 to 0.5 mg per cm² of skin..

The aqueous formulation is topically applied to the affected area in an amount of 0.1 to 6 ml, more preferably 0.3 to 3 ml and most preferably 0.5 to 1 ml.

In a preferred embodiment, the treatment comprises topically administering the aqueous formulation according to the invention to the affected skin at least once a day, more preferably at least twice a day, even more preferably at least three times a day.

In a second aspect, the present invention relates to a skin applicator comprising the aqueous formulation according to the invention, wherein the skin applicator comprises a dispenser or holder comprising the aqueous formulation, the dispenser or holder preferably being selected from a plastic flask, spray tube, a roll-on, aerosol can or soaked wipes or pads. Preferably, the skin applicator is a spray or an aerosol tube comprising the aqueous formulation.

The suitable holder or dispenser according to the invention efficiently enables the subject to apply the ipratropium formulation to the affected area of the skin, such as the underarm, hand palms, feet soles, neck, back, etc., where control of perspiration and/or deodorancy is desired.

In a third aspect, the present invention relates to a method of preparing an aqueous formulation according to the invention, the method comprising the steps of combining ipratropium with a dermatologically acceptable pH adjustment agent to produce an aqueous formulation having a pH in the range of 3.0 to 5.0.

In one embodiment of the invention, the pH adjustment agent is present in the aqueous phase before adding the ipratropium thereto. In another embodiment of the invention the pH adjustment agent is combined with the ipratropium in the solid form, followed by combining the mixture of solids to an aqueous solution.

A preferred method according to the invention comprising combining ipratropium with the pH adjustment agent (e.g. citric acid and sodium citrate) in the solid form, followed by combining (e.g. solubilising) the solids mixture with an aqueous solution. Preferably, the solubilisation step is performed at 21 °C, but not more than 30 °C.

Optionally, the aqueous solution further comprises dermatologically acceptable excipients. The dermatologically acceptable excipients may be added to the aqueous solution before or after adding the ipratropium thereto.

If necessary, the pH of the final aqueous formulation can be adjusted by adding a pH neutralizer or acidifier until the pH in the range of 3.0 to 6.0 is achieved.

A forth aspect of the invention relates to a non-therapeutic method for cosmetically reducing sweating and/or skin odours, the method comprising applying the aqueous formulation according to the invention or obtainable by the method of the invention to the affected skin.

Preferably, the non-therapeutic method according to the invention comprises applying the aqueous formulation as a deodorant or antiperspirant, preferably in an affected area(s) selected from armpits, face, back, palm or feet soles.

In a particularly preferred embodiment, the non-therapeutic method according to the invention comprises applying the aqueous formulation to the affected area(s) at least once a day, preferably twice a day, even more preferred at least three times a day.

Preferably, the non-therapeutic method according to the invention provides a dry touch on the skin of a subject suffering from excessive perspiration.

The invention will now be illustrated by the following non-limiting examples

### EXAMPLES

### Example 1

Two aqueous formulations were prepared. Formulation 1 contained 0.06% (w/v) ipratropium (11637 Sigma-Aldrich Ipratropium bromide monohydrate >98% (TLC), powder). Formulation 2 contained 0.12% (w/v) ipratropium (11637 Sigma-Aldrich Ipratropium bromide monohydrate >98% (TLC), powder). A placebo aqueous formulation (P) was also prepared. All aqueous formulations (including P) contained citrate buffer solution (pH 4, 10 mM) and benzalkonium chloride (0.01%) as a preservative.

The formulations were prepared following the steps of:
- combining 0,01% benzalkonium chloride with demineralized water to provide an aqueous solution of 0.1 %(w/v) benzalkonium chloride;
- providing weighted amounts of ipratropium bromide, citric acid and sodium citrate in the powder form;
- dissolving powders into the benzalkonium solution under stirring;
- measuring the pH at 21 °C using pH measurement strips;
- adjusting the final volume of the aqueous solution; and
- adjusting the pH of the final solution to pH 4.0 with HCI or NaOH solution, if necessary.

All solutions were kept in white polyethylene flacons with a suitable mechanical spraying device. The final formulations were stored for 6 weeks at 21 °C.

Formulations 1 and 2 were stable in the storage conditions for at least 6 weeks without visual changes.

### Example 2

The antiperspirant efficacy of the formulations prepared as described in Example 1 was examined by using patients clinically diagnosed with hyperhidrosis.

Each patient was subjected to a double-blind, placebo controlled test for a period of 7 consecutive days for each of the test formulations. The subjects were asked to apply the formulation twice a day, two sprays each armpit (1 spray = about 0,15 ml solution).

Each subject was asked to evaluate the axillary odor in a scale from 1 to 4 following the Hyperhidrosis Disease Severity Scale (HDSS) as determined by the International Hyperhidrosis Society (https://www.sweathelp.org/pdf/HDSS.pdf):

### How would you rate the severity of your hyperhidrosis?

1. My sweating is never noticeable and never interferes with my daily activities
2. My sweating is tolerable but sometimes interferes with my daily activities
3. My sweating is barely tolerable and frequently interferes with my daily activities
4. My sweating is intolerable and always interferes with my daily activities

The results were measured as a reduction in HDSS score (in percentage), *i.e.*, HDSS for a certain solution and a certain volunteer in relation to a basic HDSS score of the same volunteer when nothing was used.

An average reduction in the HDSS of 52% was reported by the tested subjects using either Formulations 1 and 2, as compared to 13% reduction when using formulation P.

### Example 3

Compositions with high amounts of gelling agent are known in the art (WO2010/062930 - Ex. 12). A comparative test of compositions without and without 3g/100g methylcellulose involves allowing subjects to use the compositions in the same fashion as described in Example 2 above. The results are also to be assessed according to the scale of Example 2. Amounts are given in g/100g.

| | Ex. 12 WO2010/062930 | Comp. A |
|---|---|---|
| Ipratropium bromide (monohydrate) | 2.59 | 2.59 |
| Methylcellulose | 3.00 | 1.00 |
| Purified water | 94.41 | 96.41 |

The composition according to Ex. 12 of WO2010/062930 presents inferior results on symptoms control of hyperhidrosis (*i.e.*, reducing sweating) in subjects.

## Claims

1. An aqueous formulation for use in the treatment of a skin disease, said treatment comprising topically administering to a human patient an aqueous formulation comprising, by weight of the total formulation:
• 0.01- 1.5 wt.% ipratropium equivalent;
• 70-98 wt.% aqueous phase;
• dermatologically acceptable pH adjustment agent to provide the formulation with a pH in the range of 3.0 to 6.0 at 21°C.

2. Aqueous formulation for use according to claim 1, wherein the ipratropium is present in an amount of 0.03 to 1.5 wt.%, preferably in an amount of 0.05 to 1.0 wt.%.

3. Aqueous formulation for use according to claim 1 or 2, wherein the formulation comprises a gelling agent.

4. Aqueous formulation for use according to claim 3, wherein the gelling agent is present in amount of at most 1 wt.%.

5. Aqueous formulation for use according to claims 3 or 4, wherein the gelling agent is a cellulose polymer, preferably selected from carboxymethyl cellulose, ethylcellulose, hydroxyethyl cellulose, hydroxypropyl cellulose, methylcellulose, or combinations thereof.

6. Aqueous formulation for use according to anyone of the preceding claims, wherein the aqueous phase comprises, based on the weight of the aqueous phase, at least 50 wt.% water.

7. Aqueous formulation for use according anyone of the preceding claims, wherein the formulation has a pH in the range of 3.5 to 5.0 at 21 °C.

8. Aqueous formulation for use according to anyone of the preceding claims, wherein the dermatologically acceptable pH adjustment agent being selected from buffering systems or salts of weak organic or inorganic acids, preferably acetate or citrate.

9. Aqueous formulation for use according to anyone of the preceding claims, wherein the formulation is a liquid aqueous solution or is an aqueous gel.

10. Aqueous formulation for use according to anyone of the preceding claims, wherein the aqueous formulation is topically administered to the affected skin at least once a day.

11. Aqueous formulation for use according to anyone of the preceding claims, wherein the skin disease is primary or secondary hyperhidrosis.

12. Aqueous formulation for use according to anyone of the preceding claims, wherein the ipratropium is administered in an amount of at least 0.005 mg per cm² of skin.

13. A skin applicator comprising the aqueous formulation for use according to claims 1 to 12, wherein the skin applicator comprises a dispenser or holder comprising the aqueous formulation, the dispenser or holder preferably being selected from a plastic flask, spray tube, a roll-on, aerosol can or soaked wipes or pads.

14. A method of preparing an aqueous formulation according to claims 1 to 12, the method comprising the steps of combining ipratropium with an aqueous phase and a gelling agent and adjusting the pH of the formulation to a pH in the range of 3.0 to 6.0 by adding a pH adjustment agent, wherein the gelling agent is present in the aqueous formulation in an amount of at most 1 wt.%.

15. A non-therapeutic method for cosmetically reducing sweating and/or skin odours, the method comprising applying the aqueous formulation according to claims 1 to 12, the skin applicator according to claim 13 or the formulation obtainable by the method according to claim 14 to the skin.
